# EUROPEAN PATENT SPECIFICATION

(11) **EP 2 564 847 B1**
(45) Date of publication and mention of the grant of the patent: **23.11.2016**
(21) Application number: 11774360.9
(22) Date of filing: 22.04.2011
(51) Int. Cl.: A61K 31/4365, A61K 9/16, A61K 9/20, A61K 9/48, A61K 47/48

(54) **PHARMACEUTICAL COMPOSITION FOR IMPROVING SOLUBILITY OF PRASUGREL AND ITS PREPARATION METHOD**
PHARMAZEUTISCHE ZUSAMMENSETZUNG ZUR VERBESSERUNG DER LÖSLICHKEIT VON PRASUGREL UND HERSTELLUNGSVERFAHREN DAFÜR
COMPOSITION PHARMACEUTIQUE POUR L'AMÉLIORATION DE LA SOLUBILITÉ DE PRASUGREL ET SON PROCÉDÉ DE PRÉPARATION

(30) Priority: 27.04.2010 CN 201010158669
(43) Date of publication of application: 06.03.2013
(73) Proprietor: Jiangsu Hansoh Pharmaceutical Group Co., Ltd., Jiangsu 222047 (CN)
(72) Inventor: CEN, Junda, Lianyungang Jiangsu 222047 (CN); ZHANG, Chunhong, Lianyungang Jiangsu 222000 (CN); ZHANG, Qi, Lianyungang Jiangsu 222000 (CN); LU, Aifeng, Lianyungang Jiangsu 222000 (CN)
(74) Representative: Nevant, Marc
(86) International application number: PCT/CN2011/073162
(87) International publication number: WO 2011/134369

(56) References cited:
- EP-A1- 2 377 520
- WO-A1-2010/094471
- WO-A1-2011/015599
- WO-A1-2011/098536
- WO-A2-2008/060934
- WO-A2-2008/060934
- CN-A- 101 456 864
- CN-A- 101 554 378
- CN-A- 101 633 662
- CN-A- 101 810 611
- US-A1- 2007 243 243
- US-A1- 2010 004 279
- DATABASE IP.COM 28 July 2010 'Formulation containing a thienopyridine antiplatelet prodrug', XP013139370 Database accession no. IPCOM000198165D

## Description

### Field of the invention

This invention relates to a pharmaceutical composition containing prasugrel or salts thereof, and to a method for preparing said composition. Specially, this invention relates to a pharmaceutical composition improving the dissolution rate of prasugrel or its salts at high pH by using surfactant technology, and a method of preparation of the pharmaceutical composition.

### Background of the invention

The chemical name of Prasugrel is 5-(2-cyclopropyl-1-(2-fluorophenyl)-2-oxoethyl)-4,5,6,7-tetrahydrothieno[3,2-c]pyridin-2-yl acetate.

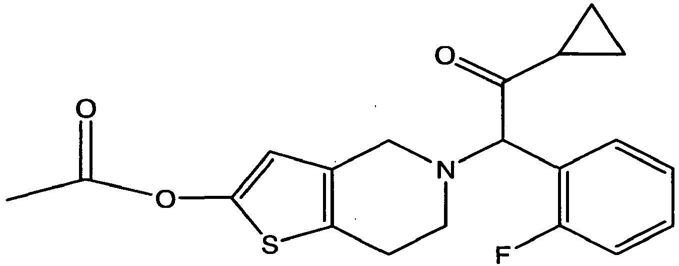

Its molecular formula is C₂₀H₂₀FNO₃S, and its molecular weight is 373.44.

Prasugrel is an oral platelet inhibitor and an anticlotting drug. It was originally developed for patients suffered from acute coronary syndrome who need percutaneous coronary intervention, including patients who need stent placement. Research results show that prasugrel could reduce the death resulting from nonfatal heart disease and stroke more effectively than clopidogrel, and obviously decrease the stent thrombosis risk.

Eli Lilly filed a patent application (US20080166893) relating to prasugrel hydrochloride in 2006, in which it claims an administration scheme. The film coated, diamond shaped tablet of 5 and 10 mg of dosage formed prasugrel hydrochloride (prasugrel, Efient) is on sale in Europe and USA currently. A single loading dosage of 60 mg, a maintenance dose of 10 mg per day was suggested, and at the same time 75∼325 mg aspirin should also be administrated every day. Prasugrel hydrochloride, which is slightly soluble in pH 1-4, very slightly soluble in pH 5 and insoluble in pH 6-7, has a higher bioavailability than its base form in gastric higher pH. According to related reports from FDA-review, the bioavailability is about equal when prasugrel hydrochloride or basic form of prasugrel is administrated alone; when prasugrel hydrochloride or basic form of prasugrel is administrated together with PPI or H2 receptor antagonists, which will bring gastric pH arising, the bioavailability of form of prasugrel is obviously lower than the prasugrel hydrochloride. According to the solubility of prasugrel hydrochloride and prasugrel, it can been seen that the solubility of prasugrel in pH 1∼7 is poorer than its hydrochloride, so improving the bioavailability of basic form of prasugrel can overcome the solubility hurdle of basic form of prasugrel in different pH condition.

WO 2008/060394 describes an oil-in-water emulsion containing a tetrahydropyridine antiplatelet agent. US 2010/004279 describes a solid medicinal preparation containing prasugrel and mannitol or lactose. US 2007/243243 describes an oral dosage form containing an antiplatelet agent and an acid inhibitor. WO 2010/0944741, WO 2011/015599, WO 2011/098536 and EP-A-2 377 520 each describe a pharmaceutical composition containing prasugrel and a surfactant.

A surfactant is an amphiphilic material having hydrophilicity and lipophilicity. The surfactant can improve the drug wettability, avoid drug particle coalescence, with the help of the micellar solubilization, so that the solubility of hydrophobic drug and the bioavailability of drug are improved. Types of surfactant can be divided into four categories: anionic surfactant, cationic surfactant, amphiphilic surfactant and non-ionic surfactant.

### Description of the invention

This invention aims to provide a pharmaceutical composition containing prasugrel or salts thereof, wherein said prasugrel or salts thereof exist in the composition in a molecular, an ionic, a crystalline or an amorphous form, wherein:
said pharmaceutical composition improves the dissolution rate of prasugrel or salts thereof at high pH;
said dissolution rate is equal to or higher than the dissolution rate of prasugrel hydrochloride;
said pH value is in the range of 1.0<pH≤7.0;
said composition contains a surfactant;
said pharmaceutical composition is a solid preparation, wherein
90% of the particles of the drug have a size less than or equal to 75µmt;
wherein said particle size is preferably less than or equal to 50µm, more preferably less than or equal to 10µm, most preferably less than or equal to 5µm; said surfactant is selected from sodium dodecyl sulfate, bile salt, tween, span, polyoxyethylene or/and poloxamer, preferably the surfactant is selected from sodium dodecyl sulfate, bile salt or/and poloxamer, more preferably the surfactant is sodium dodecyl sulfate; wherein the weight ratio of said prasugrel or salts thereof to the surfactant is less than or equal to 1:2, preferably less than or equal to 1:10, more preferably less than or equal to 1:20; said solid preparation contains fillers, and said filler is selected from mannitol, starch, modified starch, microcrystalline cellulose, lactose or/and calcium hydrophosphate, and is preferably a combination of mannitol and microcrystalline cellulose; said solid preparation contains a lubricant, said lubricant is selected from metallic stearates, stearic acid, hydrogenated vegetable oil, talcum powder or/and colloidal silicon dioxide, and is preferably a combination of colloidal silicon dioxide and magnesium stearate; wherein based on the total weight of the solid preparation being 100%, the weight ratio of said colloidal silicon dioxide is 0%-5%, preferably 0%-3%, more preferably 0%-2%, and the weight ratio of said magnesium stearate is 0.5%-1%.

After being micronized solely, the dissolution rate of prasugrel at pH 4.5 and pH 6.8 conditions is improved, but there is still a gap, compared to that of a micronized preparation of prasugrel hydrochloride.

The purposes of this invention are achieved through the following steps:
Adding a surfactant into the pharmaceutical composition

After being micronized solely, compared to the dissolution rate of the preparation of prasugrel hydrochloride, the dissolution rate of prasugrel is improved obviously, but at pH6.8 conditions the solubility of prasugrel is poorer than that of prasugrel hydrochloride. The dissolution rate of micronized prasugrel at higher pH can be equal to or higher than that of the preparation of micronized prasugrel hydrochloride by adding a certain amount of a surfactant.

The surfactant is well known by one skilled in the art, and is divided into anionic surfactant such as sodium dodecyl sulfate and bile salt and non-ionic surfactant such as Tween, Span, polyoxyethylene and poloxamer. In order to improve the solubility of prasugrel, the surfactant is preferably selected from sodium dodecyl sulfate, bile salt and poloxamer, more preferably selected from sodium dodecyl sulfate and bile salt, and most preferably is sodium dodecyl sulfate.

In this invention, the particle size of the drug was studied also. The study found that the smaller particle size, the more beneficial to the dissolution rate of prasugrel. The drug particle size is less than or equal to 75µm, preferably less than or equal to 50µm, more preferably less than or equal to 10µm, more preferably less than or equal to 5µm.

In this invention the amount of the surfactant was studied also. The study found that when the weight ratio of prasugrel to the surfactant is less than or equal to 1:2, preferably less than or equal to 1:10, more preferably less than or equal to 1:20, the better solubility of prasugrel is obtained.

The filler is selected from mannitol, starch, modified starch, microcrystalline cellulose, lactose or/and calcium hydrophosphate, preferably selected from a combination of mannitol and microcrystalline cellulose.

The lubricant is selected from metallic stearates, stearic acid, hydrogenated vegetable oil, talcum powder or/and colloidal silicon dioxide, preferably selected from a combination of colloidal silicon dioxide and magnesium stearate. The weight ratio of said colloidal silicon dioxide is 0%-5%, preferably 0%-3%, more preferably 0-2%. The weight ratio of magnesium stearate is 0.5%-1%.

The preparation technology can be dry-granulation technology. All the ingredients including the active ingredient and the surfactant except the lubricant are pre-mixed together, the mixture is screened by a sieve and compressed, the lubricant is added, and then the mixture is compressed into tablets or directly filled into capsules. When the surfactant is oily or semisolid, the surfactant can be mixed with microcrystalline cellulose or silica (aerosil). After sieving, the mixture is mixed with prasugrel, then dry-granulation is carried out.

The preparation technology can be wet-granulation. All the ingredients including active ingredient and the surfactant except for the lubricant are mixed together, the mixture is wetted with purified water and dried, or the surfactant is added into purified water as a wetting agent, then the mixture is granulated and dried. After mixing with the lubricant, the mixture is compressed into tablets or filled into capsules.

The preparation technology can also be direct tableting method. All the ingredients except for the lubricant are mixed together. After sieving and mixed uniformly, the lubricant is added, and the mixture is compressed directly into the tablets or filled into the capsules.

In this invention the prasugrel and hydrophilic carrier material were used as starting material, to improve the dissolution rate of the prasugrel or its salts at high pH by adding surfactants thereby to improve the bioavailability of prasugrel base at high pH, specially this invention has the following advantages:
The bioavailability of prasugrel at high pH is improved by adding surfactants, and the dissolution rate of prasugrel is similar to or higher than that of prasugrel hydrochloride at high pH.

### Preferred embodiments

The invention will be illustrated specifically by following embodiments, the embodiments of this invention are used to illustrate technical scheme of this invention, no limitations to spirit and scope of the invention are intended.

### Example 1

**Pharmaceutical composition:**

| | |
|---|---|
| Drug (prasugrel) | 5g |
| Sodium dodecyl sulfate | 10g |
| Mannitol | 73g |
| Microcrystalline cellulose | 50g |
| Cross-linked carboxymethyl cellulose sodium | 7.5g |
| Hydroxypropyl methyl cellulose | 3g |
| Magnesium stearate | 1.5g |

### Preparation Process:

The drug (prasugrel) was micronized, mixed with sodium dodecyl sulfate, microcrystalline cellulose, mannitol, crosslinked sodium carboxymethyl cellulose and hydroxypropyl methyl cellulose were added successively through equal increment, the mixture was screened 5 times through a 60 mesh sieve, magnesium stearate (pretreated by screening through a 60 mesh sieve) was added last, the mixture was mixed uniformly, and then compressed directly to get the tablets.

### Example 2

**Pharmaceutical composition:**

| | |
|---|---|
| Drug (prasugrel) | 5g |
| Sodium dodecyl sulfate | 5g |
| Mannitol | 71.5g |
| Microcrystalline cellulose | 50g |
| Cross-linked carboxymethyl cellulose sodium | 7.5g |
| Hydroxypropyl methyl cellulose | 3g |
| Aerosil | 1.5g |
| Magnesium stearate | 1.5g |

### Preparation Process:

The drug (prasugrel) was micronized, mixed with other excipients except for the surfactant and the lubricant. Sodium dodecyl sulfate was dissolved in purified water according to the prescription and the solution was used as a wetting agent. The wetting agent was added to the mixed excipients to prepare a damp mass. The damp mass was screened through 30 mesh sieve to granulate and dried. Magnesium stearate was added after the mixture was screened through a 40 mesh sieve, and then the mixture was compressed to get the tablets.

### Example 3

**Pharmaceutical composition:**

| | |
|---|---|
| Drug (prasugrel) | 5g |
| Poloxamer | 5g |
| Mannitol | 70g |
| Microcrystalline cellulose | 50g |
| Cross-linked carboxymethyl cellulose sodium | 7.5g |
| Hydroxypropyl methyl cellulose | 3g |
| Aerosil | 3g |
| Magnesium stearate | 1.5g |

### Preparation Process:

The drug (prasugrel) was micronized, mixed with other excipients except for the surfactant and the lubricant. Poloxamer was dissolved in purified water according to the prescription, it was used as a wetting agent. The wetting agent was added to the mixed excipients to prepare a damp mass. The damp mass was screened through a 30 mesh sieve to get granules and dried. Magnesium stearate was added after the mixture was screened through a 40 mesh sieve, and then the mixture was compressed to get the tablets.

### Test example 1

PVP was used as the carrier material, the solid dispersion was prepared by the solvent method (drug:carrier=1:10), the method to evaluate solubility *in vitro* is given as follows:
(1) Calculate and weigh solid dispersion which contains the drug substance (prasugrel) (5mg), according to the method of solubility test (Appendix XC, the fist method), 1000ml pH4.5 phosphate buffer was used as dissolution medium, the rotation speed was 50 rpm. According to the method, 5ml of the solution was taken at 5min, 10min, 15min, 20min, 30min, 45min and 60min (5ml dissolution medium was supplemented after the 5 ml solution was taken out) and filtered. The filtrate was taken as the test solution, determined by HPLC.
(2) Calculate and weigh solid dispersion which contains the drug substance (5mg), according to the method of dissolution rate test (Appendix XC, the fist method), 1000ml pH6.8 phosphate buffer was used as dissolution medium, the rotation speed was 50 rpm. According to the method, 5ml of the solution was taken at 5min, 10min, 15min, 20min, 30min, 45min and 60min (5ml dissolution medium was supplemented after the 5 ml solution was taken out) and filtered. The filtrate was taken as the test solution, determined by HPLC.
(3) Control prescription of drug (prasugrel hydrochloride) was prepared as follows:

| | |
|---|---|
| prasugrel hydrochloride | 5.49g |
| Mannitol | 79.51g |
| Microcrystalline cellulose | 50g |
| Cross-linked carboxymethyl cellulose sodium | 7.5g |
| Hydroxypropyl methyl cellulose | 3g |
| Aerosil | 3g |
| Magnesium stearate | 1.5g |

### Preparation Process:

The control drug (prasugrel hydrochloride) was micronized, added successively with microcrystalline cellulose, mannitol, cross-linked carboxymethyl cellulose sodium and hydroxypropyl methyl cellulose and mixed. The mixture was screened 5 times through a 60 mesh sieve. Magnesium stearate (pretreated by screening through a 60 meshe sieve) was added last, the mixture was mixed uniformly, and compressed directly to get the tablets.

The dissolution rate of examples 1-3 were evaluated by the evaluation system mentioned above, the results of the dissolution rate showed that the dissolution rate of prasugrel solid preparation was improved significantly. Comparative data for the dissolution rate of the products in example 1 and the control drug (prasugrel hydrochloride) tablet at pH4.5 and pH6.8 are shown below.

**The dissolution rate was evaluated at pH4.5 phosphate buffer:**

**Table 1: Prasugrel hydrochloride vs product of example 1**

| | 0min | 5min | 10min | 15min | 20min | 30min |
|---|---|---|---|---|---|---|
| Prasugrel hydrochloride | 0 | 16.71 | 26.66 | 32.45 | 36.78 | 43.42 |
| Product of example 1 | 0 | 25.73 | 53.76 | 56.93 | 59.15 | 60.28 |

**The dissolution rate was evaluated at pH6.8 phosphate buffer:**

**Table 2: Prasugrel hydrochloride vs product of example 1**

| | 0min | 5min | 15min | 30min | 45min | 60min |
|---|---|---|---|---|---|---|
| Prasugrel hydrochloride | 0 | 13.69 | 21.03 | 23.99 | 25.13 | 25.62 |
| Product of example 1 | 0 | 17.31 | 24.92 | 29.57 | 34.32 | 36.97 |

## Claims

1. A pharmaceutical composition containing prasugrel or one of its salts, wherein said prasugrel or salts thereof exist in the composition in a molecule, an ion, a crystalline or an amorphous form, wherein said pharmaceutical composition is a solid preparation and contains a surfactant, wherein the weight ratio of prasugrel or salts thereof to the surfactant is less than or equal to 1:2, wherein 90% of the particles of prasugrel or salts thereof have a size less than or equal to 75µm, and wherein said pharmaceutical composition improves the dissolution rate of prasugrel or salts thereof at high gastric pH.

2. The pharmaceutical composition of claim 1, wherein said dissolution rate is equal to or higher than the solubility of prasugrel hydrochloride.

3. The pharmaceutical composition of claim 1, wherein said pH value is in the range of 1.0<pH≤7.0.

4. The pharmaceutical composition of claim 1, wherein said surfactant is sodium dodecyl sulfate.

5. The pharmaceutical composition of claim 1, wherein said solid preparation is **characterized in that** 90% of the particles of prasugrel or salts thereof have a size less than or equal to 50µm.

6. The pharmaceutical composition of claim 1, wherein said solid preparation is **characterized in that** 90% of the particles of prasugrel or salts thereof have a size less than or equal to 10µm.

7. The pharmaceutical composition of claim 1, wherein said solid preparation is **characterized in that** 90% of the particles of prasugrel or salts thereof have a size less than or equal to 5µm.

8. The pharmaceutical composition of claim 1, wherein said surfactant is selected from bile salt, tween, span, polyoxyethylene or/and poloxamer.

9. The pharmaceutical composition of claim 1, wherein the weight ratio of prasugrel or salts thereof to the surfactant is less than or equal to 1:10, preferably less than or equal to 1:20.

10. The pharmaceutical composition of claim 1, wherein said solid preparation contains a filler.

11. The pharmaceutical composition of claim 10, wherein said filler is selected from mannitol, starch, modified starch, microcrystalline cellulose, lactose or/and calcium hydrophosphate.

12. The pharmaceutical composition of claim 11, wherein said filler is a combination of mannitol and microcrystalline cellulose.

13. The pharmaceutical composition of claim 1, wherein said solid preparation contains a lubricant.

14. The pharmaceutical composition of claim 13, wherein said lubricant is selected from metallic stearates, stearic acid, hydrogenated vegetable oil, talcum powder or/and colloidal silicon dioxide.

15. The pharmaceutical composition of claim 14, wherein said lubricant is a combination of colloidal silicon dioxide and magnesium stearate.

16. The pharmaceutical composition of claim 14, wherein based on the total weight of the solid preparation being 100%, the weight ratio of said colloidal silicon dioxide is 0%-5%

17. The pharmaceutical composition of claim 16, wherein based on the total weight of the solid preparation being 100%, the weight ratio of said colloidal silicon dioxide is 0%-3%.

18. The pharmaceutical composition of claim 16, wherein based on the total weight of the solid preparation being 100%, the weight ratio of said colloidal silicon dioxide is 0%-2%.

19. The pharmaceutical composition of claim 14, wherein in relation to the total weight of the solid preparation being 100%, the weight ratio of said magnesium stearate is 0.5%-1%.

## Patentansprüche

1. Pharmazeutische Zusammensetzung enthaltend Prasugrel oder eines seiner Salze, wobei das Prasugrel oder die Salze davon in der Zusammensetzung in einem Molekül, einem Ion, einer kristallinen oder einer amorphen Form vorliegen, wobei die pharmazeutische Zusammensetzung eine feste Zubereitung ist und ein Tensid enthält, wobei das Gewichtsverhältnis von Prasugrel oder den Salzen davon zu dem Tensid weniger als oder gleich 1:2 beträgt, wobei 90 % der Teilchen des Prasugrel oder der Salze davon eine Größe von weniger als oder gleich 75 µm aufweisen, und wobei die pharmazeutische Zusammensetzung die Auflösungsrate von Prasugrel oder den Salzen davon bei einem hohen gastrischen pH-Wert verbessert.

2. Pharmazeutische Zusammensetzung nach Anspruch 1, wobei die Auflösungsrate gleich der oder höher als die Löslichkeit von Prasugrelhydrochlorid ist.

3. Pharmazeutische Zusammensetzung nach Anspruch 1, wobei der pH-Wert in dem Bereich von 1,0 < pH ≤ 7,0 liegt.

4. Pharmazeutische Zusammensetzung nach Anspruch 1, wobei das Tensid Natriumdodecylsulfat ist.

5. Pharmazeutische Zusammensetzung nach Anspruch 1, wobei die feste Zubereitung **dadurch gekennzeichnet ist, dass** 90 % der Teilchen des Prasugrels oder der Salze davon eine Größe von weniger als oder gleich 50 µm aufweisen.

6. Pharmazeutische Zusammensetzung nach Anspruch 1, wobei die feste Zubereitung **dadurch gekennzeichnet ist, dass** 90 % der Teilchen des Prasugrels oder der Salze davon eine Größe von weniger als oder gleich 10 µm aufweisen.

7. Pharmazeutische Zusammensetzung nach Anspruch 1, wobei die feste Zubereitung **dadurch gekennzeichnet ist, dass** 90 % der Teilchen des Prasugrels oder der Salze davon eine Größe von weniger als oder gleich 5 µm aufweisen.

8. Pharmazeutische Zusammensetzung nach Anspruch 1, wobei das Tensid aus Gallensalz, Tween, Span, Polyoxyethylen oder/und Poloxamer ausgewählt ist.

9. Pharmazeutische Zusammensetzung nach Anspruch 1, wobei das Gewichtsverhältnis von Prasugel oder den Salzen davon zu dem Tensid weniger als oder gleich 1:10 beträgt, vorzugsweise weniger als oder gleich 1:20.

10. Pharmazeutische Zusammensetzung nach Anspruch 1, wobei die feste Zubereitung einen Füllstoff enthält.

11. Pharmazeutische Zusammensetzung nach Anspruch 10, wobei der Füllstoff aus Mannitol, Stärke, modifizierter Stärke, mikrokristalliner Cellulose, Lactose oder/und Calciumhydrophosphat ausgewählt ist.

12. Pharmazeutische Zusammensetzung nach Anspruch 11, wobei der Füllstoff eine Kombination aus Mannitol und mikrokristalliner Cellulose ist.

13. Pharmazeutische Zusammensetzung nach Anspruch 1, wobei die feste Zubereitung ein Schmiermittel enthält.

14. Pharmazeutische Zusammensetzung nach Anspruch 13, wobei das Schmiermittel aus metallischen Stearaten, Stearinsäure, hydrogeniertem Pflanzenöl, Talkumpuder oder/und kolloidalem Siliciumdioxid ausgewählt ist.

15. Pharmazeutische Zusammensetzung nach Anspruch 14, wobei das Schmiermittel eine Kombination aus kolloidalem Siliciumdioxid und Magnesiumstearat ist.

16. Pharmazeutische Zusammensetzung nach Anspruch 14, wobei das Gewichtsverhältnis des kolloidalen Siliciumdioxids 0-5 % in Bezug auf das Gesamtgewicht der festen Zubereitung, das 100 % entspricht, beträgt.

17. Pharmazeutische Zusammensetzung nach Anspruch 16, wobei das Gewichtsverhältnis des kolloidalen Siliciumdioxids 0-3 % in Bezug auf das Gesamtgewicht der festen Zubereitung, das 100 % entspricht, beträgt.

18. Pharmazeutische Zusammensetzung nach Anspruch 16, wobei das Gewichtsverhältnis des kolloidalen Siliciumdioxids 0-2 % in Bezug auf das Gesamtgewicht der festen Zubereitung, das 100 % entspricht, beträgt.

19. Pharmazeutische Zusammensetzung nach Anspruch 14, wobei das Gewichtsverhältnis des Magnesiumstearats 0,5-1 % in Bezug auf das Gesamtgewicht der festen Zubereitung, das 100 % entspricht, beträgt.

## Revendications

1. Composition pharmaceutique contenant du prasugrel ou l'un de ses sels, dans laquelle le prasugrel ou ses sels existent dans la composition sous la forme d'une molécule, d'un ion, d'une forme cristalline ou amorphe, laquelle composition pharmaceutique est une préparation solide et contient un tensioactif, dans laquelle le rapport en poids du prasugrel ou de ses sels au tensioactif est inférieur ou égal à 1:2, dans laquelle 90 % des particules de prasugrel ou de ses sels ont une taille inférieure ou égale à 75 µm, et laquelle composition pharmaceutique améliore le taux de dissolution du prasugrel ou de ses sels à un pH gastrique élevé.

2. Composition pharmaceutique selon la revendication 1, dans laquelle, ledit taux de dissolution est égal ou supérieur à la solubilité du chlorhydrate de prasugrel.

3. Composition pharmaceutique selon la revendication 1, dans laquelle ladite valeur de pH est située dans la plate 1,0 < pH ≤ 7,0.

4. Composition pharmaceutique selon la revendication 1, dans laquelle ledit tensioactif est le dodécylsulfate de sodium.

5. Composition pharmaceutique selon la revendication 1 dans laquelle ladite préparation solide est **caractérisée en ce que** 90 % des particules de prasugrel ou de ses sels ont une taille inférieure ou égale à 50 µm.

6. Composition pharmaceutique selon la revendication 1, dans laquelle ladite préparation solide est **caractérisée en ce que** 90 % des particules de prasugrel ou de ses sels ont une taille inférieure ou égale à 10 µm.

7. Composition pharmaceutique selon la revendication 1, dans laquelle ladite préparation solide est **caractérisée en ce que** 90 % des particules de Prasugrel ou de ses sels ont une taille inférieure ou égale à 5 µm.

8. Composition pharmaceutique selon la revendication 1, dans laquelle ledit tensioactif est choisi parmi un sel biliaire, un Tween, un Span, un polyoxyéthylène et/ou un Poloxamer.

9. Composition pharmaceutique selon la revendication 1, dans laquelle le rapport en poids du prasugrel ou de ses sels au tensioactif est inférieur ou égal à 1:10, de préférence inférieur ou égal à 1:20.

10. Composition pharmaceutique selon la revendication 1, dans laquelle ladite préparation solide contient une charge.

11. Composition pharmaceutique selon la revendication 10, dans laquelle, ladite charge est choisie parmi le mannitol, l'amidon, l'amidon modifié, la cellulose microcristalline, le lactose et/ou l'hydrophosphate de calcium.

12. Composition pharmaceutique selon la revendication 11, dans laquelle ladite charge est une combinaison de mannitol et de cellulose microcristalline.

13. Composition pharmaceutique selon la revendication 1, dans laquelle ladite préparation solide contient un lubrifiant.

14. Composition pharmaceutique selon la revendication 13, dans laquelle ledit lubrifiant est choisi parmi les stéarates métalliques, l'acide stéarique, l'huile végétale hydrogénée, la poudre de talc et/ou le dioxyde de silicium colloïdal.

15. Composition pharmaceutique selon la revendication 14, dans laquelle ledit lubrifiant est une combinaison de dioxyde de silicium colloïdal et de stéarate de magnésium.

16. Composition pharmaceutique selon la revendication 14, dans laquelle, par rapport au poids total de la préparation solide qui est de 100 %, la proportion en poids dudit dioxyde de silicium colloïdal est de 0 % à 5 %.

17. Composition pharmaceutique selon la revendication 16, dans laquelle, par rapport au poids total de la préparation solide qui est de 100 %, la proportion en poids dudit dioxyde de silicium colloïdal est de 0 % à 3 %.

18. Composition pharmaceutique selon la revendication 16, dans laquelle, par rapport au poids total de la préparation solide qui est de 100 %, la proportion en poids dudit dioxyde de silicium colloïdal est de 0 % à 2 %.

19. Composition pharmaceutique selon la revendication 14, dans laquelle, par rapport au poids total de la préparation solide qui est de 100 %, la proportion en poids dudit stéarate de magnésium est de 0,5 % à 1 %.
